# EUROPÄISCHE PATENTSCHRIFT

(11) **EP 2 126 041 B1**
(45) Veröffentlichungstag und Bekanntmachung des Hinweises auf die Patenterteilung: **11.04.2012**
(21) Anmeldenummer: 08707478.7
(22) Anmeldetag: 31.01.2008
(51) Int. Cl.: C12M 3/08

(54) **VORRICHTUNG UND VERFAHREN ZUR MECHANISCHEN VEREINZELUNG VON ZELLEN AUS EINEM ZELLVERBUND**
DEVICE AND METHOD FOR THE MECHANICAL DECOLLATION OF CELLS FROM A CELL COMPOSITE
PROCÉDÉ ET DISPOSITIF DE SÉPARATION MÉCANIQUE DE CELLULES D'UN ENSEMBLE DE CELLULES

(30) Priorität: 02.02.2007 DE 102007005369
(43) Veröffentlichungstag der Anmeldung: 02.12.2009
(73) Patentinhaber: EPPENDORF AG, 22339 Hamburg (DE)
(72) Erfinder: LINK, Holger, 22339 Hamburg (DE); PAPRA, Alexander, 22159 Hamburg (DE); BLUMENTRITT, Michael, 22083 Hamburg (DE); DUONG, Vinh, 22850 Norderstedt (DE)
(74) Vertreter: Hauck Patent- und Rechtsanwälte
(86) Internationale Anmeldenummer: PCT/EP2008/000791
(87) Internationale Veröffentlichungsnummer: WO 2008/092697

(56) Entgegenhaltungen:
- WO-A-01/85902
- WO-A-99/02958
- DE-A1- 2 645 622
- DE-A1- 3 218 079
- US-A- 4 028 190
- US-A- 5 879 939

## Beschreibung

### 1. Gebiet der Erfindung

Die vorliegende Erfindung betrifft Vorrichtungen und ein Verfahren zur Vereinzelung von Zellen aus einem Zellverbund mit Hilfe eines Scherrotors.

### 2. Hintergrund der Erfindung

Für unterschiedlichste Versuchszwecke ist es erforderlich, in einem Zellverbund vorliegende Zellen zu vereinzeln. Der Stand der Technik bietet zu diesem Zweck unterschiedliche Verfahren an. Zu diesen Verfahren zählen beispielsweise die Ultraschallbehandlung eines Zellverbunds gemäß US 5,879,939, die Mehrfachfiltration durch Filter unterschiedlicher Porengrössc gemäß US 5,888,409, das Verkleinern oder Schreddern eines Zcllverbunds gemäß US 4,028,190 oder die Scherung einer flüssigen Probe mit Zellverbund mittels Pipettieren oder eines Scherrotors gemäß DE 32 18 079., die Abtrennung lebensfähiger Zellen aus Zellsuspensionen unter Verwendung von Hydrozyklonen gemäß WO 01/85902 A1 oder die Abtrennung von Anhäufungen biologischer Zellen gemäß DE 3218079 A1 durch einen Rotor.

Die oben genannten Verfahren haben verschiedene Nachteile. Zum einen wird häufig der in der flüssigen Probe vorhandene Zellverbund durch den Energieeintrag zur Vereinzelung der Zellen überbelastet. Dies führt zu einer Zerstörung der Zellen und zu einer Verlängerung des Präparationsverfahrens, bis intakte vereinzelte Zellen vorliegen. Der obige Nachteil tritt gerade bei mechanischen Vereinzelungsverfahren auf, wie dem oben genannten Verkleinerer oder beim Pipettieren der flüssigen Probe.

Der Scherrotor gemäß DE 32 18 079 hat den Nachteil, dass eine flüssige Probe mit Zellverbund überall den gleichen Scherkräften ausgesetzt ist. Zudem wird die Probe durch turbulente Strömungen innerhalb des Scherrotors bewegt, so dass eine gleichmäßige Probenbearbeitung nicht gewährleistet ist.

Das Verfahren zur Abtrennung lebensfähiger Zellen aus einer Zellsuspension gemäß WO 01/85902 A1 betrifft mindestens ein Hydrozyklon, dem über eine Einlasseinrichtung tangential zum Zylinderraum eine Zellsuspension zur Abtrennung von Zellen zugeführt wird. Das Prinzip der Abtrennung von Zellen basiert hierbei auf das Zusammenwirken von Flich- und Strömungskräften, die bedingt durch die Konstruktion eines Hydrozyklons in unterschiedliche Richtung zeigen, sodass eine mit Zellen angereicherte und eine mit Zellen abgereicherte Suspension entsteht. Die mit Zellen abgereicherte Suspension fließt über eine zentrale Überlauf-Auslasseinrichtung, dem Wirbelsucher, und die mit Zellen angereicherte Suspension über eine Unterlauf-Auslasseinrichtung ab. Das Hydrozyklon hat den Nachteil, dass zum Erreichen einer gewünschten Ab- oder Anreichcrungkonzentration mehrere Hydrozyklone in Reihe oder parallel geschaltet werden müssen, was mit einem erhöhten Materialaufwand einhergeht und somit unökonomisch ist.

Die Vorrichtung für die Auftrennung von Anhäufungen biologischer Zellen gemäß DE 3218079 A1 betrifft einen drehbar im Inneren eines Bechers angeordneten, bevorzugt einen reinen oder mit innerer Aushöhlung oder schraubenlinienförmig zylindrischen, aus Plastik bestehenden Rotor, welcher Scherkräfte zur Auftrennung der Anhäufung der Zellen in der sich im Becher befindende Suspension erzeugt.

Dabei ist der Rotor fest an einen elektrischen Motor über eine Welle angekoppelt und sowohl Becher als auch Rotor haben beide bevorzugt zylindrische Gestalt, wobei eine konische Konfiguration ebenfalls möglich ist. Nachteilig hierbei ist, dass die Auftrennung unterschiedlicher Zellsuspensionen nur erschwert möglich ist, da der Rotor fest am Motor über ein Welle angekoppelt ist und somit zuvor gereinigt werden muß, statt den Rotor einfach durch einen anderen auszutauschen.

Es ist daher die Aufgabe der vorliegenden Erfindung, eine Vorrichtung und ein Verfahren zur Vereinzelung von Zellen aus einem Zellverbund bereitzustellen, mit denen im Vergleich zum Stand der Technik Proben verlässlicher verarbeitet werden können und für unterschiedliche, zu verarbeitende Suspensionen einfacher funktionsbereit hergerichtet werden kann.

### 3. Zusammenfassung der Erfindung

Die obige Aufgabe wird durch Vorrichtungen gemäß den unabhängigen Patentansprüchen 1, 8, 10 und 11 und durch ein Verfahren gemäß dem Patentanspruch 17 gelöst. Vorteilhafte Ausgestaltungen und Weiterentwicklungen der vorliegenden Erfindung gehen aus der folgenden Beschreibung, den Zeichnungen und den anhängenden Patentansprüchen hervor.

Die vorliegende Erfindung offenbart eine Vorrichtung zur mechanischen Vereinzelung von Zellen aus einem Zellverbund, insbesondere einen Scherrotor, die die folgenden Merkmale aufweist: einen Rotor mit einer Rotorwand, der konzentrisch in einem Gefäß angeordnet ist, eine mit einem Motor verbundene Rotoraufnahme, an der der Rotor lösbar befestigbar und mit der eine Drehbewegung des Motors auf den Rotor übertragbar ist, während sich der Rotor in seiner Längsrichtung auf einen Boden des Gefäßes zu verjüngt, so dass unterschiedliche Umfangsgeschwindigkeiten des Rotors über die Rotorwand auf eine flüssige Probe in dem Gefäß übertragbar sind.
Der oben beschriebene Scherrotor umfasst ein Gefäß, in dem eine flüssige Probe mit Zellverbund aufgenommen wird. Innerhalb dieses Gefäßes ist drehbar ein Rotor angeordnet, so dass durch die Drehung des Rotors innerhalb des Gefäßes der Zellverbund in der flüssigen Probe geschert und in einzelne Zellen vereinzelt wird. Des Weiteren wird durch die Geometrie des Rotors gewährleistet, dass eine Zirkulation der flüssigen Probe innerhalb des Gefäßes und somit auch innerhalb des Spalts zwischen Rotorwand und Gefäßwand stattfindet. Auf diese Weise wird das gesamte Probenvolumen bearbeitet und das Bilden von Rückständen, beispielsweise am Boden des Gefäßes, verhindert. Zu diesem Zweck weist der Rotor eine sich nach unten verjüngende, bevorzugt kegelförmige, Struktur auf. Bei Drehung des Rotors bewegen sich auf Grund dieser Geometrie zwei auf der Rotorwand gedachte Punkte unterschiedlich schnell, sofern diese mit einer unterschiedlichen Entfernung zum Boden des Gefäßes angeordnet sind. Basierend auf diesen unterschiedlichen Rotationsgeschwindigkeiten, die mit zunehmender Entfernung vom Boden des Gefäßes zunehmen, ergeben sich Druckunterschiede im Gefäß und Spalt zwischen Rotorwand und Gefäßwand, die zu einer vorteilhaften Zirkulation und Scherung der flüssigen Probe führen.

Gemäß unterschiedlicher Ausführungsformen weist das Gefäß eine zylindrische Gestalt oder eine Gestalt geringerer Konizität verglichen mit dem kegelförmigen Rotor auf oder weitet sich in Richtung des Bodens des Gefäßes auf.

Gemäß einer weiteren Ausführungsform sind Rotor und/oder Gefäß aus Einwegmaterial, wie beispielsweise Kunststoff oder PVC hergestellt, um diese nach Bearbeitung einer flüssigen Probe entsorgen zu können. Die Verwendung von Rotor und/oder Gefäß aus Einwegmaterial erfordert, dass der Rotor mit geringem Aufwand, beispielsweise automatisch an der Rotoraufnahme befestigbar und von dieser lösbar ist. Zu diesem Zweck weist die Rotoraufnahme ein Außengewinde und der Rotor ein zu dem Außengewinde passendes Innengewinde auf. Gemäß einer weiteren Alternative umfasst die Rotoraufnahme einen Vorsprung und der Rotor einen dazu passenden Schnappverschluss, so dass der Rotor ebenfalls lösbar an der Rotoraufnahme befestigbar ist. Im Falle des einfachen Verbindens von Rotoraufnahme und Rotor über einen Schnappverschluss umfasst die Rotoraufnahme zudem eine Drehsperre, mit der auf Grundlage einer formschlüssigen und/oder kraftschlüssigen Verbindung die Drehbewegung der Rotoraufnahme auf den Rotor übertragbar ist.

Es ist zudem bevorzugt, die obige Vorrichtung mit technischen Hilfsmitteln zur Durchführung einer Trübungsmessung der flüssigen Probe im Gefäß auszustatten. Zu diesem Zweck wird das Gefäß aus einem zumindest teilweise durchstrahlbaren Material hergestellt. Gemäß einer Alternative ist dieses Material optisch durchstrahlbar oder weist eine Mehrzahl von Fenstern auf, so dass mit Hilfe einer Lichtquelle und eines entsprechenden Sensors zur Erfassung einer Lichtmenge die Trübungsmessung an der flüssigen Probe durchführbar ist. Es ist des Weiteren denkbar, die Trübungsmessung mittels Ultraschall durchzuführen, so dass das Gefäß aus für Ultraschall durchlässigem Material hergestellt sein muss. Die Trübungsmessung wird beispielsweise durch eine Steuereinheit oder einen Computer durchgeführt und überwacht und die dabei erfassten Daten werden entsprechend ausgewertet.

Die vorliegende Erfindung offenbart des Weiteren eine Zellisolationseinheit zur mechanischen Vereinzelung von Zellen aus einem Zellverbund, die die folgenden Merkmale aufweist: einen Rotor mit einer Rotorwand, der automatisch und bewegbar in einer Mehrzahl von Gefäßen mit einer Gefäßwand anordenbar ist, eine mit einem Motor verbundene Rotoraufnahme, an der der Rotor lösbar befestigbar und mit der eine Drehbewegung des Motors auf den Rotor übertragbar ist, während die Mehrzahl von Gefäßen in einer Gefäßhalterung angeordnet ist, so dass über eine automatische Bewegung des Rotors und/oder der Gefäßhalterung der Rotor konzentrisch in jeweils einem der Gefäße der Gefäßhalterung positionierbar und drehbar ist.

Die oben beschriebene Zellisolationseinheit eröffnet die Möglichkeit, eine Mehrzahl flüssiger Proben mit Zellverbund systematisch hintereinander mit dem erfindungsgemäßen Scherrotor zu bearbeiten. Die verschiedenen flüssigen Proben sind in unterschiedlichen Gefäßen innerhalb einer Gefäßhalterung angeordnet. Vorteilhafter Weise ist die Anordnung der Gefäße regelmäßig, so dass beispielsweise über ein Steuermodul oder eine Computersteuerung jede einzelne Gefäßposition gezielt anfahrbar ist. Sobald der Rotor entweder über eine Eigenbewegung, eine Bewegung der Gefäßhalterung oder über eine kombinierte Bewegung von Rotoraufnahme und Gefäßhalterung innerhalb des ausgewählten Gefäßes konzentrisch angeordnet ist, kann eine Bearbeitung der flüssigen Probe mit Zellverbund in dem ausgewählten Gefäß erfolgen.

Gemäß einer ersten Alternative weist der Rotor der Zellisolationseinheit eine zylindrische Form auf. Es ist des Weiteren bevorzugt, dass sich der Rotor in seiner Längsrichtung auf den Boden des Gefäßes zu verjüngt, insbesondere kegelförmig ausgebildet ist. Diese unterschiedlichen Rotorgestaltungen sind mit Gefäßen kombinierbar, die jeweils nur an ihrer Oberseite eine Öffnung zum Befüllen der Gefäße mit einer flüssigen Probe aufweisen. Gemäß verschiedener Ausführungsformen besitzen die Gefäße eine zylindrische oder eine sich zum Boden des Gefäßes verjüngende, insbesondere kegelförmige, Gestalt. Es ist des Weiteren denkbar, das Gefäß derart auszubilden, dass es sich in Richtung seines Bodens aufweitet.

Gemäß einer weiteren Ausführungsform ist die Gefäßhalterung als Kreisport ausgebildet, indem die Mehrzahl von Gefäßen entlang einer kreisförmigen Bahn gleichmäßig beabstandet in angepassten Öffnungen entfernbar angeordnet sind. Es ist des Weiteren denkbar, die Gefäßhalterung als eckigen Port auszubilden, so dass die Mehrzahl von Gefäßen entlang gerader Linien, beispielsweise äquidistant voneinander beabstandet, angeordnet sind. Die festgelegte Geometrie der Gefäßhalterung gibt eine genaue Position der einzelnen Gefäße vor, so dass der Rotor automatisch, beispielsweise computergesteuert, mit geringem Aufwand in den individuellen Gefäßen positionierbar ist. Des Weiteren eröffnet eine derartige Konstruktion die Möglichkeit, unterschiedliche Gefäße mit unterschiedlichen Proben gezielt zu befüllen und/oder unterschiedliche Gefäße mit speziell angepassten Vereinzelungsverfahren zu bearbeiten.

Gemäß einer weiteren Ausführungsform umfasst die Zellisolationseinheit eine Austauschvorrichtung, mit der Rotor und/oder Gefäße entfernbar und durch jeweils einen neuen Rotor und/oder ein neues Gefäß ersetzbar sind. Eine derartige Austauschvorrichtung bildet die Voraussetzung für eine weitere Automatisierung der Verarbeitung einer Mehrzahl flüssiger Proben, wenn diese beispielsweise durch eine automatische Dosiervorrichtung den neu eingesetzten Gefäßen zugeführt werden.

Es ist des Weiteren bevorzugt, die Zellisolationseinheit mit einer Temperiereinheit auszustatten, so dass eine flüssige Probe in einem oder einer Mehrzahl der Gefäße in der Gefäßhalterung gezielt temperierbar ist.

Die vorliegende Erfindung offenbart zudem ein Vereinzelungsverfahren von Zellen aus einem Zellverbund, das die folgenden Schritte aufweist: Einbringen einer flüssigen Probe mit Zellverbund in ein Gefäß, Anordnen eines sich in seiner Längsrichtung auf einen Boden des Gefäßes zu verjüngenden Rotors konzentrisch in dem Gefäß derart, dass unterschiedliche Umfangsgeschwindigkeiten des Rotors über eine Rotorwand auf eine flüssige Probe in dem Gefäß übertragbar sind, und Drehen des Rotors derart, dass der Zellverbund in Zellen vereinzelt wird. Gemäß einer bevorzugten Alternative des obigen Vereinzelungsverfahrens werden eine Drehgeschwindigkeit des Rotors in dem Gefäß und eine Geometrie des Rotors derart aufeinander abgestimmt, dass die flüssige Probe innerhalb des Spalts während der Drehung des Rotors zirkuliert und geschert wird. Dies gewährleistet eine Bearbeitung der gesamten Probe und verhindert nicht bearbeitete Rückstände der flüssigen Probe beispielsweise am Boden des Gefäßes.

### 4. Beschreibung der begleitenden Zeichnungen

Die vorliegende Erfindung wird unter Bezugnahme auf die begleitende Zeichnung näher erläutert. Es zeigen:
- Figur 1: eine schematische Schnittdarstellung des erfindungsgemäßen Scherrotors,
- Figur 2 A, B: jeweils eine perspektivische Darstellung einer Ausführungsform, des erfindungsgemäßen Scherrotors,
- Figur 3: eine Ausführungsform der Zellisolatinseinheit und
- Figur 4: eine weitere Ausführungsform der Zellisolationseinheit.

### 5. Detaillierte Beschreibung bevorzugter Ausführungsformen

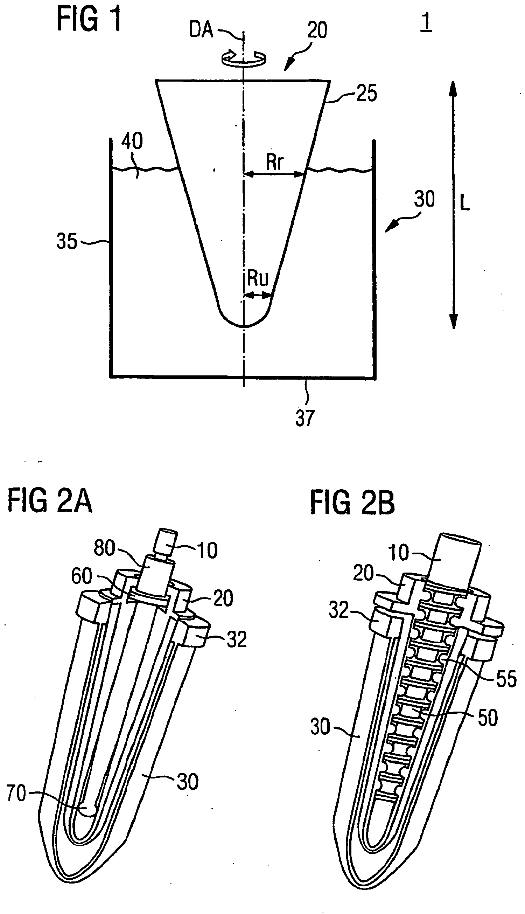

Figur 1 zeigt eine schematische Darstellung einer Ausführungsform des erfindungsgemäßen Scherrotors 1. Der Scherrotor 1 umfasst ein Gefäß 30 und einen Rotor 20, der konzentrisch und drehbar im Gefäß 30 angeordnet ist. Das Gefäß 30 weist bevorzugt eine zylindrische Innenkontur auf, während der Boden 37 des Gefäßes 30 konisch oder halbkugelförmig ausgebildet ist. Es ist ebenfalls bevorzugt, dass das Gefäß 30 kegelförmig ausgebildet ist oder eine sich in Richtung des Bodens 37 aufweitende Form aufweist. Der Rotor 20 ist an einer Rotoraufnahme 10 (nicht gezeigt, siehe unten) lösbar befestigt. Die Rotoraufnahme 10 wiederum ist mit einem Motor 90 (nicht gezeigt) verbunden, so dass eine Drehbewegung des Motors 90 auf den Rotor 20 übertragbar ist.

Der Rotor 20 wird umfänglich durch eine Rotorwand 25 begrenzt. Die Rotorwand 25 ist von einer als Strich-Punkt-Linie dargestellten Drehachse DA des Rotors 20 um den Radius R beabstandet. In Richtung seiner Längsachse L und auf den Boden 37 des Gefäßes 30 zu laufend verjüngt sich der Rotor 20. Gemäß einer bevorzugten Alternative ist der Rotor 20 kegelförmig ausgebildet. Durch die verjüngte Form des Rotors 20 ist die Rotorwand 25 in größerer Entfernung vom Boden 37 um den Radius R_{O} von der Drehachse DA beabstandet, welcher größer ist als ein Radius R_{U} in geringerer Entfernung vom Boden 37.

Wird nun der Rotor 20 durch den Motor um die Drehachse DA gedreht, bewegt sich ein Punkt auf der Rotorwand 25 mit dem Abstand R_{O} von der Drehachse DA schneller als ein Punkt auf der Rotorwand 25 mit dem Abstand R_{U} von der Drehachse DA. Durch die höhere Umfangsgeschwindigkeit im oberen Bereich des Rotors 20 im Vergleich zu seinem verjüngten Bereich entsteht eine dynamische Druckverteilung in der flüssigen Probe 40 im Gefäß 30. Durch diese Druckverteilung werden die Anteile der flüssigen Probe 40 nahe dem Boden 37 des Gefäßes 30 in den Spalt zwischen Rotorwand 25 und Gefäßwand 35 in Richtung Flüssigkeitsoberfläche bewegt bzw. gesaugt. Innerhalb des Spalts zwischen Rotorwand 25 und Gefäßwand 35 ist die flüssige Probe 40 mit Zellverbund Scherkräften ausgesetzt, die zu einer Vereinzelung der Zellen führen. Teile der flüssigen Probe 40 nahe der Gefäßwand 35 sinken wieder zum Boden 37 ab, so dass sich eine Zirkulation der flüssigen Probe 40 ergibt. Werden Drehgeschwindigkeit und Geometrie des Rotors 20 aufeinander abgestimmt, sind Zirkulation und Scherung der flüssigen Probe 40 im Gefäß 30 gezielt einstellbar. Dies gewährleistet, dass die flüssige Probe 40 auch in einem Gefäß 30 mit nur einer oberen Öffnung in den Spalt zwischen Gefäßwand 35 und Rotorwand 25 bewegt wird, ohne dass das Gefäß 30 einen Zulauf oder eine Druckzufuhr nahe des Bodens 37 aufweist. Zudem werden ausreichende Scherkräfte in der flüssigen Probe 40 mit Hilfe der planen Rotor- 25 und Gefäßwand 35 erzeugt, um die Zellen zu vereinzeln. Ergänzend ist es denkbar, die Rotorwand 25 mit Vorsprüngen und/oder Profilierungen auszubilden, um die flüssige Probe 40 zu bearbeiten.

Der Rotor 20 und das Gefäß 30 sind bevorzugt aus Kunststoff, insbesondere PVC, hergestellt. Zudem sind Rotor 20 und Gefäß 30 als Einweg- oder Mehrwegartikel realisierbar, die automatisch oder manuell austauschbar sind.

Gemäß einer Ausführungsform besteht das Gefäß 30 aus einem mit Ultraschallwellen oder Licht durchstrahlbaren Material, um an der flüssigen Probe 40 im Gefäß 30 eine Trübungsmessung durchführen zu können. Durch das durchstrahlbare Material wird Ultraschall oder Licht auf die flüssige Probe 40 gerichtet. Die reflektierten Ultraschallwellen werden beispielsweise durch den zuvor als Ultraschallquelle genutzten Ultraschallkopf erfasst und in ein elektrisches Signal umgewandelt. Dieses elektrische Signal wird zu Auswertezwecken beispielsweise einem Computer oder einer Recheneinheit zugeführt. Wird die Trübungsmessung optisch durchgeführt, strahlt man Licht durch die Gefäßwand 35 in die flüssige Probe 40 ein und erfasst nachfolgend mit Hilfe eines Sensors das Auftreten des Streulichts oder den Anteil des Durchlichts oder die in der flüssigen Probe 40 stattfindende Lichtabsorption. In Abhängigkeit von dem im Sensor erfassten Lichtsignal wird ein elektrisches Signal generiert, das wiederum an eine Auswerteeinheit weitergeleitet wird. Diese Auswerteeinheit generiert dann ein entsprechendes Signal, welches das Ergebnis der Trübungsmessung repräsentiert. Das Ergebnis der Trübungsmessung gibt Auskunft über die Vereinzelung von Zellen und/oder die Durchmischung der flüssigen Probe 40 im Gefäß 30.

Es ist ebenfalls denkbar, an Stelle eines durchstrahlbaren Materials des Gefä-βes 30 mindestens ein Fenster im Gefäß 30 vorzusehen. Mit Hilfe dieses mindestens einen Fensters, oder mit zwei einander gegenüberliegenden Fenstern ist dann die oben beschriebene Trübungsmessung durchführbar.

Gemäß einer weiteren Ausführungsform ist das Gefäß 30 mit einer Temperiereinheit verbunden. Diese erzeugt in der flüssigen Probe 40 die gewünschte Temperatur, so dass die gewünschten Versuchsbedingungen erzielt werden.

Figur 2 zeigt bevorzugte Ausführungsformen des Rotors 20, der Rotoraufnahme 10 und des Gefäßes 30. Das Gefäß 30 weist eine kegelförmige Gestalt mit einem oberen Rand 32 auf. Der Rand 32 dient dem Abstützen und Stabilisieren des Gefäßes 30 in einer Halterung beispielsweise in einer Öffnung eines Kreisports, wie er unten näher erläutert ist. Basierend auf dieser Geometrie des Gefäßes 30 ist es ohne Aufwand in einer Gefäßhalterung oder einem Kreisport (siehe unten) manuell oder automatisch positionierbar und wieder entnehmbar.

Gemäß der in Figur 2A dargestellten ersten Alternative der Rotoraufnahme 10 umfasst diese einen umlaufenden Vorsprung 60, der als Schnappverriegelung für den Rotor 20 dient. Der Rotor 20 weist einen komplementär zum Vorsprung 60 geformten elastischen Randbereich oder Schnappverschluss 80 auf, der mit dem Vorsprung 60 kraft- und/oder formschlüssig zusammenwirkt. Damit die Drehbewegung des mit der Rotoraufnahme 10 verbundenen Motors auf den Rotor 20 übertragbar ist, umfasst die Rotoraufnahme 10 eine Drehsperre 70. Diese Drehsperre 70 besitzt die Form einer Verdickung, wie in Figur 2A angedeutet ist. Diese Drehsperre 70 verklemmt sich kraftschlüssig und/oder formschlüssig im Inneren des Rotors 20, beispielsweise in einer dafür vorgesehenen Ausbuchtung oder Vertiefung. Auf diese Weise nimmt die Rotoraufnahme 10 über die Drehsperre 70 den Rotor 20 mit, so dass dieser die Drehbewegung des Motors ausführt.

Gemäß einer weiteren in Figur 2B dargestellten Ausführungsform umfasst die Rotoraufnahme 10 ein Außengewinde 55. Passend zum Außengewinde 40 umfasst der Rotor 20 ein Innengewinde 50. Der Rotor 20 ist daher mit geringem Aufwand auf die Rotoraufnahme 10 aufschraubbar.

Basierend auf den oben beschriebenen Ausführungsformen der Rotoraufnahme 10 und des Rotors 20 ist somit der Rotor 20 manuell oder automatisch an der Rotoraufnahme 10 befestigbar und von dieser entfernbar.

Die vorliegende Erfindung offenbart des Weiteren eine Zellisolationseinheit zur mechanischen Vereinzelung von Zellen aus einem Zellverbund. Diese Zellisolationseinheit stellt eine Komponente zur automatischen Dissoziierung von flüssigen Proben 40 mit Zellverbund dar. Es sind weitere, hier nicht dargestellte Komponenten denkbar, die mit der Zellisolationseinheit kombiniert werden können, um eine automatisierte Probenverarbeitung bereitzustellen. Diese Komponenten dienen beispielsweise dem automatischen Aufnehmen, Abgeben und Dosieren von Reagenzien und flüssigen Proben, dem Temperieren und Mischen der Proben und dem Separieren der Probenbestandteile, beispielsweise durch Zentrifugieren.

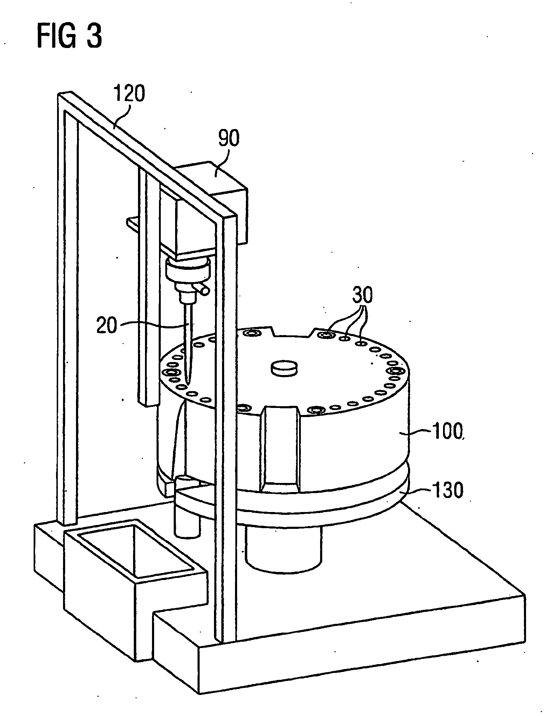

Gemäß einer ersten in Figur 3 dargestellten Ausführungsform umfasst die Zellisolationseinheit den bereits oben beschriebenen Rotor 20, der über die Rotoraufnahme mit einem Motor 90 verbunden ist. Der Motor 90 mit Rotoraufnahme und Rotor 20 ist entlang einer Führung 120 zumindest in vertikaler Richtung automatisch bewegbar. Es ist weiterhin denkbar, dass der Motor 90 auch horizontal bewegbar vorgesehen ist. Über die vertikale Bewegung des Rotors 20 entlang der Führung 120 ist der Rotor 20 in einzelne Gefäße 30 absenkbar, um dort, wie oben beschrieben, angeordnet zu sein und eine Vereinzelung von Zellen in einer flüssigen Probe durchzuführen.

Eine Mehrzahl der Gefäße 30 der Zellisolationseinheit ist in einer Gefäßhalterung 100 regelmäßig oder unregelmäßig angeordnet. Die Gefäßhalterung 100 ist bevorzugt als Kreisport ausgebildet, in dem die Gefäße 30 entlang einer kreisförmigen Bahn in voneinander gleichmäßig beabstandeten Öffnungen angeordnet sind. Die Öffnungen sind an die Gefäße 30 angepasst, so dass die Gefäße 30 mit geringem Aufwand in die Öffnungen einsetzbar und aus diesen entfernbar sind. Die Gefäße 30 weisen gemäß unterschiedlicher Alternativen eine zylindrische, eine kegelförmige oder eine sich zum Boden 37 des Gefäßes 30 hin aufweitende Form auf. Gemäß einer ersten Ausführungsform besitzt der Rotor eine zylindrische Form mit den Befestigungsmöglichkeiten, wie sie oben bezüglich des Rotors 20 beschrieben worden sind. Gemäß einer weiteren Ausführungsform wird der oben beschriebene Rotor 20 eingesetzt.

Es ist zudem bevorzugt, den Kreisport 100 auf einer motorisch bewegbaren Plattform 130 anzuordnen. Mit Hilfe der Plattform 130 ist der Kreisport 100 schrittweise bewegbar, so dass einzelne Gefäße 30 gezielt in Bezug auf den Rotor 20 positionierbar sind. Gemäß einer weiteren Alternative dient die Plattform 130 als Zentrifugiereinheit, die den Kreisport zum Separieren der Bestandteile der flüssigen Probe 40 in Drehungen versetzt. Gemäß einer weiteren Ausführungsform ist die Plattform 130 auch vertikal bewegbar. Dies eröffnet die Möglichkeit, dass Rotor 20 und Motor 90 fest angeordnet sind, während der Rotor 20 über die vertikale und die Drehbewegung des Kreisports 100 in dem jeweils gewünschten Gefäß 30 positioniert wird. Diese Positionierung kann ebenfalls über eine kombinierte Bewegung von Rotor 20/Motor 90 und Kreisport 100/Plattform 130 erfolgen.

Gemäß einer weiteren Ausführungsform umfasst die Zellisolationseinheit eine Austauschvorrichtung, mit der der verwendete Rotor 20 und die Gefäße 30 automatisch austauschbar sind. Diese Austauschvorrichtung entfernt automatisch den Rotor 20 von der Rotoraufnahme 10 und legt ihn in einem Auffangbehälter für benutzte Einwegartikel oder für zu reinigende Mehrwegartikel ab. Nachfolgend wird ein neuer Rotor 20 automatisch an der Rotoraufnahme 10 befestigt. In gleicher Weise wird automatisch ein benutztes Gefäß 30 entfernt und durch ein neues ersetzt.

Gemäß einer weiteren Alternative wird in Verbindung mit der Gefäßhalterung 100 eine Temperiereinheit (nicht gezeigt) eingesetzt. Diese Temperiereinheit bringt die flüssige Probe 40 in zumindest einem der Gefäße 30 der Gefäßhalterung 100 auf die gewünschte Temperatur, um die gewünschten Verarbeitungsbedingungen der flüssigen Probe 40 zu erzielen.

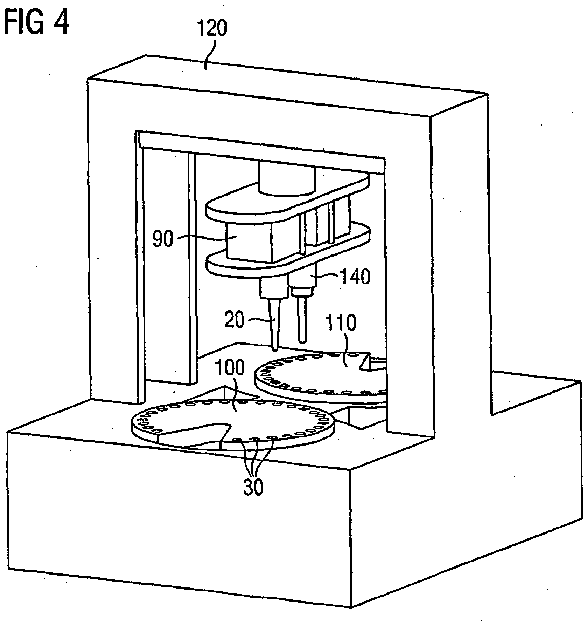

Eine weitere Ausführungsform der Zellisolationseinheit ist in Figur 4 dargestellt. Im Unterschied zur Ausführungsform der Figur 3 sind die Gefäßhalterung 100 und eine Zentrifugiereinheit 110 nebeneinander angeordnet. Entlang einer Führung 120 ist der Motor 90 mit Rotor 20 automatisch bewegbar angeordnet. Parallel zum Motor 90 ist eine Probenübertragungseinheit 140 vorgesehen. Diese Probenübertragungseinheit 140 ist bevorzugt um die vertikal verlaufende Befestigung einer Führung 120 drehbar, so dass der Rotor 20 oder die Probenübertragungseinheit 140 automatisch über einem ausgewählten Gefäß 30 im Kreisport 100 oder in der Zentrifugiereinheit 110 positionierbar ist. Die flüssigen Proben in den Gefäßen 30 im Kreisport 100 werden schrittweise unter den Rotor 20 bewegt. Der Rotor 20 wird wiederum über eine vertikale Bewegung im Gefäß 30 angeordnet und über den Motor 90 gedreht. Auf diese Weise erzielt man die Vereinzelung eines zuvor in einer flüssigen Probe enthaltenen Zellverbunds. Führt man an dieser Stelle eine Trübungsmessung (siehe oben) an der bearbeiteten flüssigen Probe im Gefäß 30 durch, erhlt man Aufschluss darüber, ob eine ausreichende Durchmischung und/oder Vereinzelung in der flüssigen Probe stattgefunden hat. In Abhängigkeit vom Ergebnis der Auswertung der Trübungsmessung wird die Behandlung der Probe fortgesetzt oder beendet. Nach erfolgter Vereinzelung wird der Rotor 20 durch die vertikale Bewegung aus dem Gefäß 30 zurückgefahren und beispielsweise durch die oben beschriebene Austauschvorrichtung (nicht gezeigt) gegen einen neuen Rotor 20 ausgetauscht. Die Probenübertragungseinheit 140 entnimmt dem Gefäß 30 die flüssige Probe mit vereinzelten Zellen und überführt sie in ein Gefäß der Zentrifugiereinheit 140. Dort wird dann beispielsweise ein Zentrifugieren der vorhandenen flüssigen Proben durchgeführt, um die unterschiedlichen Bestandteile dieser flüssigen Proben voneinander zu separieren.

### Bezugszeichenliste

- 1: Vorrichtung
- 10: Rotoraufnahme
- 20: Rotor
- 25: Rotorwand
- 30: Gefäß
- 32: Vorsprung
- 35: Gefäßwand
- 37: Boden
- 40: flüssige Probe
- 50: Innengewinde des Rotors
- 55: Außengewinde der Rotoraufnahme
- 60: Vorsprung
- 70: Drehsperre
- 80: Schnappverschluss
- 90: Motor
- 100: Gefäßhalterung
- 110: Zentrifugiereinheit
- 120: Führung
- 130: Plattform
- L: Längsrichtung
- DA: Drehachse des Rotors
- R_{O}: Radius
- R_{U}: Radius

## Patentansprüche

1. Vorrichtung (1) zur mechanischen Vereinzelung von Zellen aus einem Zcllverbund, insbesondere einem Scherrotor, die die folgenden Merkmale aufweist:
a. einen Rotor (20) mit einer Rotorwand (25), der konzentrisch in einem Gefäß (30) angeordnet ist,
b. eine mit einem Motor verbundene Rotoraufnahme (10), an der der Rotor (20) lösbar befestigbar und mit der eine Drehbewegung des Motors auf den Rotor (20) übertragbar ist, während
c. sich der Rotor (20) in seiner Längsrichtung (L) auf einen Boden (37) des Gefäßes (30) zu verjüngt, so dass unterschiedliche Umfangsgeschwindigkeiten des Rotors (20) über die Rotorwand (25) auf eine flüssige Probe in dem Gefäß (30) übertragbar sind.

2. Vorrichtung (1) gemäß Anspruch 1, deren Rotor (20) kegelförmig ausgebildet ist.

3. Vorrichtung (1) gemäß einem der vorhergehenden Ansprüche, deren Rotoraufnahme (10) ein Außengewinde (55) und deren Rotor (20) ein zu dem Außengewinde (40) passendes Innengewinde (50) aufweist.

4. Vorrichtung (1) gemäß Anspruch 1 oder 2, deren Rotoraufnahme (10) einen Vorsprung (60) und deren Rotor (20) einen Schnappverschluss (80) aufweist, so dass der Rotor (20) lösbar an der Rotoraufnahme (10) befestigbar ist.

5. Vorrichtung (I) gemäß Anspruch 4, deren Rotoraufnahme (10) eine Drehsperre (70) umfasst, mit der auf Grundlage einer formschlüssigen und/oder kraftschlüssigen Verbindung die Drehbewegung der Rotoraufnahme (10) auf den Rotor (20) übertragbar ist.

6. Vorrichtung (1) gemäß einem der vorhergehenden Ansprüche, die des Weiteren eine Gefäßbefestigung aufweist, mit der das Gefäß (30) lösbar befestigbar ist.

7. Vorrichtung (1) gemäß einem der vorhergehenden Ansprüche, deren Gefäß (30) aus einem zumindest teilweise durchstrahlbaren Material besteht oder eine Mehrzahl von Fenstern aufweist und die eine Licht oder Ultraschallquelle und einen Sensor umfasst, so dass eine Trübungsmessung einer Flüssigkeit im Gefäß (30) durchführbar ist.

8. Rotor (20) zur mechanischen Vereinzelung von Zellen aus einem Zellverbund, der die folgenden Merkmale aufweist:
a. einen sich in Längsrichtung (L) verjüngenden, insbesondere kegelförmigen, Grundkörper mit einer Befestigungsanordnung (80; 50),
b. so dass der Rotor (20) drehfest an einer Rotoraufnahme (10) einer Vorrichtung (1) zur mechanischen Vereinzelung von Zellen aus einem Zellverbund befestigbar ist.

9. Rotor (20) gemäß Anspruch 8, dessen Befestigungsanordnung ein Innengewinde (50) im Grundkörper oder einen Schnappverschluss (80) an einer offenen Seite des Grundkörper aufweist, so dass der Rotor (20) lösbar befestigbar ist.

10. Gefäß (30) zur mechanischen Vereinzelung von Zellen aus einem Zellverbund, das die folgenden Merkmale aufweist:
a. einen kegelförmigen oder zylindrischen Hohlkörper zur Aufnahme eines sich in Längsrichtung auf einen Boden des Gefäßes (30) zu verjüngenden Rotors, der in seiner Längsrichtung (L) an einer Seiten offen und an der gegenüberliegenden Seite geschlossen ist, während
b. das Gefäß (30) aus einem durchstrahlbaren Material besteht oder eine Mehrzahl von Fenstern umfasst, und c. eine Halteanordnung, mit der das Gefäß (30) in einer Vorrichtung (1) zur mechanischen Vereinzelung von Zellen aus einem Zcllvcrbund lösbar befestigbar ist.

11. Zellsolationseinheit zur mechanischen Vereinzelung von Zellen aus einem Zellverbund, die die folgenden Merkmale aufweist:

12. Zellisolationseinheit gemäß Anspruch 11, deren Rotor (20) eine sich in seiner Längsrichtung (L) auf den Boden (37) des Gefäßes (30) zu verjüngende Gestalt aufweist, insbesondere kegelförmig ausgebildet ist.
a. einen Rotor (20) mit einer Rotorwand (25), der automatisch und bewegbar in einer Mehrzahl von Gefäßen (30) mit einer Gefäßwand (35) anordenbar ist und sich in Längsrichtung (L) auf einen Boden des Gefäßes (30) zu verjüngt,
b. eine mit einem Motor (90) verbundene Rotoraufnahme (10), an der der Rotor (20) lösbar befestigbar und mit der eine Drehbewegung des Motors (90) auf den Rotor (20) übertragbar ist, während
c. die Mehrzahl von Gefäßen (30) in einer Gefäßhalterung (100) angeordnet ist, so dass über eine automatische Bewegung des Rotors (20) und/oder der Gefäßhalterung der Rotor (20) konzentrisch in jeweils einem der Gefäße (30) der Gefäßhalterung positionierbar ist.

13. Zellisolationseinheit gemäß einem der Ansprüche 11 bis 12, deren Gefäßhalterung (100) als Kreisport ausgebildet ist, in dem die Mehrzahl von Gefäßen (30) entlang einer kreisförmigen Bahn gleichmäßig beabstandet in angepassten Öffnungen entfernbar angeordnet sind.

14. Zellisolationseinheit gemäß Anspruch 13, deren Kreisport (100) schrittweise bewegbar ist, so dass einzelne Gefäße (30) gezielt in Bezug auf den Rotor (20) positionierbar sind.

15. Zellisolationseinheit gemäß einem der Ansprüche 11 bis 14, deren Rotor (20) und/oder Gefäß (30) mit Hilfe einer Austauschvorrichtung automatisch entfernbar und durch jeweils einen neuen Rotor (20) und/oder ein neues Gefäß (30) ersetzbar ist.

16. Zellisolationseinheit gemäß einem der Ansprüche 11 bis 15, die eine Temperiereinheit aufweist, mit der eine Flüssigkeit in zumindest einem der Gefäße (30) gezielt temperierbar ist.

17. Vereinzelungsverfahren von Zellen aus einem Zellverbund, das die folgenden Schritte aufweist:
a. Einbringen einer flüssigen Probe mit Zellverbund in ein Gefäß (30),
b. Anordnen eines sich in seiner Längsrichtung (L) auf einen Boden (37) des Gefäßes (30) zu verjüngenden Rotors (20) konzentrisch in dem Gefäß (30) derart, dass unterschiedliche Umfangsgeschwindigkeiten des Rotors (20) über eine Rotorwand (25) auf eine flüssige Probe in dem Gefäß (30) übertragbar sind, und
c. Drehen des Rotors (20) derart, dass der Zellverbund in Zellen vereinzelt wird.

18. Vereinzelungsverfahren gemäß Anspruch 17, mit dem weiteren Schritt: Abstimmen einer Drehgeschwindigkeit des Rotors (20) in dem Gefäß (30) und einer Geometrie des Rotors (20) derart aufeinander, dass die flüssige Probe innerhalb des Gefäßes (30) während der Drehung des Rotors (20) zirkuliert.

## Claims

1. A device (1) for the mechanical decollation of cells from a cell composite, in particular, a shear rotor, which has the following features :
a a rotor (20) with a rotor wall (25), that is concentrically disposed in a receptacle (30),
b. a rotor seat (10), connected to a motor, to which the rotor (20) is fastened in detachable manner, and with which a rotational movement of the motor can be transmitted to the rotor (20), while
c. the rotor (20) tapers in its longitudinal direction (L) towards the bottom (37) of the receptacle (30), so that different circumferential speeds of the rotor (20) can be transmitted via the rotor wall (25) to a liquid sample in the receptacle (30).

2. The device (1) according to Claim 1, whose rotor (20) is designed cone-shaped.

3. The device (1) according to one of the preceding claims, whose rotor seat (10) has an outside thread (50) and whose rotor (20) has an inside thread (55) matched to the outside thread (50).

4. The device (1) according to Claim 1 or 2, whose rotor seat (10) has a projection (60), and whose rotor (20) has a snap fit (80), so that the rotor (20) can be fastened to the rotor seat (10) in a detachable manner.

5. The device (1) according to Claim 4, whose rotor seat (10) includes a rotational interlock (70), with which on the basis of a positive lock or non-positive lock connection, the rotational movement of the rotor seat (10) can be transmitted to the rotor (20).

6. The device (1) according to one of the preceding claims, which also has a receptacle holder, with which the receptacle (30) can be fastened in a detachable manner.

7. The device (1) according to one of the preceding claims, whose receptacle (30) is composed of an at least partially radiation permeable material, or has a plurality of windows, and which includes a light source or ultrasound source and a sensor, so that a turbidity measurement of a liquid in the receptacle (30) can be performed.

8. A rotor (20) for the mechanical decollation of cells from a cell composite, which has the following features:
a. a base body that tapers in the longitudinal direction (L), in particular conc-shaped, with an attachment arrangement (80; 50),
b. so that the rotor (20) can be fastened, rotationally fixed to a rotor seat (10) of a device (1) for mechanical decollating of cells from a cell composite.

9. The rotor (20) according to Claim 8, whose attachment arrangement has an inside thread (50) in the base body or a snap fit (80) on an open side of the base body, so that the rotor (20) can be fastened in a detachable manner.

10. A receptacle (30) for the mechanical decollation of cells from a cell composite, which has the following features:
a. a cone-shaped or cylindrical hollow body, which in its longitudinal direction (L) is open on one side and is closed on the opposite side, for receiving a rotor, which tapers in its longitudinal direction (L) towards a bottom of the receptacle (30), while
b. the receptacle (30) is composed of a radiation permeable material, or has a plurality of windows.

11. A cell isolation unit for the mechanical decollation of cells from a cell composite, which has the following features:
a. a rotor (20) with a rotor wall (25), where said rotor can be disposed automatically and movably in a plurality of receptacles (30) with a receptacle wall (35) and where said rotor tapers in its longitudinal direction (L) towards a bottom of the receptacle (30);
b. a rotor seat (10), connected to a motor (90), to which the rotor (20) can be attached in a detachable manner, and with which a rotational movement of the motor (90) can be transmitted to the rotor (20), while
c. the plurality of receptacles (30) is disposed in a receptacle holder (100) so that via an automatic movement of the rotor (20) and/or the receptacle holder, the rotor (20) can be positioned concentrically in each of the receptacles (30) of the receptacle holder.

12. The cell isolation unit according to Claim 11, whose rotor (20) has a shape that tapers in its longitudinal direction (L) towards the bottom (37) of the receptacle (30), in particular, is designed cone-shaped.

13. The cell isolation unit according to one of the Claims 11 or 12, whose receptacle holder (100) is designed as a circular holder, in which the plurality of receptacles (30) is disposed removably, equidistant in matched openings along a circular track.

14. The cell isolation unit according to Claim 13, whose circular holder (100) can be moved in a step-wise manner so that individual receptacles (30) can be positioned in a directed manner in reference to the rotor (20).

15. The cell isolation unit according to one of the Claims 11 to 14, whose rotor (20) and/or receptacle (30) can be automatically removed using an exchange device, and can be replaced, respectively, by a new rotor (20) and/or a new receptacle (30).

16. The cell isolation unit according to one of the Claims 11 to 15, which has a temperature control unit with which the liquid in at least one of the receptacles (30) can be temperature controlled.

17. A method for decollating cells from a cell composite, which has the following steps:
a. introducing of a liquid sample with a cell composite into a receptacle (30),
b. concentrically disposing of a rotor (20) that tapers in its longitudinal direction
(L) towards a bottom (37) of the receptacle (30), into the receptacle (30), so that different circumferential speeds of the rotor (20) can be transmitted via a rotor wall (25) to a liquid sample in the receptacle (30), and
c. rotating the rotor (20) so that the cell composite is decollated into cells.

18. The method for decollation according to Claim 17, with the further step:
matching a rotational speed of the rotor (20) in the receptacle (30) and the geometry of the rotor (20) to each other so that the liquid sample circulates within the receptacle (30) during the rotation of the rotor (20).

## Revendications

1. Dispositif de séparation mécanique de cellules d'un ensemble de cellules, en particulier d'un rotor de cisaillement, qui présente les caractéristiques suivantes :
a. un rotor (20) avec une paroi de rotor (25) qui est disposé de façon concentrique dans un récipient (30),
b. un logement de rotor (10) raccordé à un moteur et auquel le rotor (20) peut être fixé de façon détachable et avec lequel un mouvement de rotation du moteur peut être communiqué au rotor (20), pendant que
c. le rotor (20) se rétrécit, dans sa direction longitudinale (L), vers un fond du récipient (30) de sorte que différentes vitesses circonférentielles du rotor (20) peuvent être communiquées via la paroi de rotor (25) à un échantillon liquide dans le récipient (30).

2. Dispositif (1) selon la revendication 1, dont le rotor (20) est constitué en forme de cône.

3. Dispositif (1) selon une des revendications précédentes, dont le logement de rotor (10) présente un filet extérieur (55) et dont le rotor (20) présente un filet intérieur (50) adapté au filet extérieur (40).

4. Dispositif (1) selon la revendication 1 ou 2, dont le logement de rotor (10) présente une saillie (60) et dont le rotor (20) présente une fermeture à déclic (80) de sorte que le le rotor (20) peut être fixé de façon détachable au logement de rotor (10).

5. Dispositif (1) selon la revendication 4, dont le logement de rotor (10) comprend un tourniquet (70) avec lequel, sur la base d'une liaison mécanique et/ou d'une liaison par friction, le mouvement de rotation du logement de rotor (10) peut être communiqué au rotor (20).

6. Dispositif (1) selon une des revendications précédentes, qui présente en plus une fixation de récipient avec laquelle le récipient (30) peut être fixé de façon détachable.

7. Dispositif (1) selon une des revendications précédentes, dont le récipient (30) se compose d'un matériau au moins partiellement perméable aux rayonnements ou bien présente une pluralité de fenêtres, et qui comprend une source de lumière ou d'ultrasons et un capteur, de sorte qu'une mesure de turbidité d'un liquide dans le récipient (30) peut être effectuée.

8. Rotor (20) pour la séparation mécanique de cellules d'un ensemble de cellules qui présente les caractéristiques suivantes :
a. un corps de base, en particulier de forme conique, se rétrécissant dans la direction longitudinale (L), avec un agencement de fixation (80 ; 50),
b. de sorte que le rotor (20) peut être fixé, bloqué en rotation, à un logement de rotor (10) d'un dispositif (1) de séparation mécanique de cellules d'un ensemble de cellules.

9. Rotor (20) selon la revendication 8, dont l'agencement de fixation présente un filet intérieur (50) dans le corps de base ou bien une fermeture à déclic (80) sur un côté ouvert du corps de base, de sorte que le rotor (20) peut être fixé de façon détachable.

10. Récipient (30) pour la séparation mécanique de cellules d'un ensemble de cellules, qui présente les caractéristiques suivantes :
a. un corps creux de forme conique ou cylindrique pour loger un rotor qui se rétrécit dans la direction longitudinale vers un fond du récipient (30), qui est ouvert sur un côté dans sa direction longitudinale (L) et qui est fermé sur le côté opposé, pendant que
b. le récipient (30) se compose d'un matériau perméable aux rayonnements ou comprend une pluralité de fenêtres et c. un agencement de retenue avec lequel le récipient (30) peut être fixé de façon détachable dans un dispositif (1) de séparation mécanique de cellules d'un ensemble de cellules.

11. Unité d'isolement de cellules pour la séparation mécanique de cellules d'un ensemble de cellules, qui présente les caractéristiques suivantes :
a. un rotor (20) avec une paroi de rotor (25) qui peut être disposé automatiquement et de façon mobile dans une pluralité de récipients (30) avec une paroi de récipient (35) et qui se rétrécit dans le direction longitudinale (L) vers un fond du récipient (30).
b. un logement de rotor (10) raccordé à un moteur (90), et auquel le rotor (20) peut être fixé de façon détachable et avec lequel un mouvement de rotation du moteur (90) peut être communiqué au rotor (20), pendant que
c. la pluralité de récipients (30) est disposée dans un élément de retenue de récipient (100) de sorte que, par le biais d'un mouvement automatique du rotor (20) et/ou de l'élément de retenue de récipient, le rotor (20) peut être positionné de façon concentrique dans respectivement un des récipients (30) de l'élément de retenue de récipient.

12. Unité d'isolement de cellules selon la revendication 11, dont le rotor (20) présente une configuration qui se rétrécit dans sa direction longitudinale (L) vers le fond (37) du récipient (30), et qui est en particulier configuré de façon conique.

13. Unité d'isolement de cellules selon une des revendications 11 à 12, dont l'élément de retenue de récipient (100) est constitué sous forme de point d'accès circulaire dans lequel la pluralité de récipients (30) sont disposés dans des ouvertures adaptées selon un espacement régulier le long d'une piste circulaire, de façon à pouvoir être enlevés.

14. Unité d'isolement de cellules selon la revendication 13, dont le point d'accès circulaire (100) est mobile pas à pas de sorte que des récipients (30) individuels peuvent être positionnés de façon ciblée par rapport au rotor (20).

15. Unité d'isolement de cellules selon une des revendications 11 à 14, dont le rotor (20) et/ou le récipient (30) peut être enlevé automatiquement à l'aide d'un dispositif de remplacement et peut être remplacé par respectivement un rotor (20) neuf et/ou par un récipient (30) neuf.

16. Unité d'isolement de cellules selon une des revendications 1 à 15, qui présente une unité d'équilibrage de température avec laquelle un liquide peut être tempéré de façon ciblée dans au moins un des récipients (30).

17. Procédé de séparation mécanique de cellules d'un ensemble de cellules, qui présente les étapes suivantes :
a. Mise en place d'un échantillon liquide avec un ensemble de cellules dans un récipient (30) ;
b. Agencement d'un rotor (20), se rétrécissant dans sa direction longitudinale (L) vers un fond (37) du récipient (30), de façon concentrique dans le récipient (30), de sorte que différentes vitesses circonférentielles du rotor (20) peuvent être communiquées, par le biais d'une paroi de rotor (25), à un échantillon liquide dans le récipient (30) ; et
c. Mise en rotation du rotor (20) de sorte que l'ensemble de cellules est séparé en cellules.

18. Procédé de séparation mécanique de cellules d'un ensemble de cellules selon la revendication 17, avec l'étape supplémentaire :
Harmonisation entre elles d'une vitesse de rotation du rotor (20) dans le récipient (30) et d'une géométrie du rotor (20) de sorte que l'échantillon liquide circule à l'intérieur du récipient (30) pendant la rotation du rotor (20).
